# EUROPEAN PATENT SPECIFICATION

(11) **EP 0 946 876 B1**
(45) Date of publication and mention of the grant of the patent: **12.01.2005**
(21) Application number: 97910335.5
(22) Date of filing: 23.09.1997
(51) Int. Cl.: G01N 33/68

(54) **MODEL FOR ATHERO/ARTERIOSCLEROSIS BASED ON PROTEOHEPARAN SULFATE**
AUF PROTEOHEPARANSULFAT BERUHENDEM MODELL FÜR ATHERO/ARTERIOSKLEROSE
MODELE BASEE SUR L'HEPARAN SULFATE POUR L'ATHERO/ARTERIOSCLEROSE

(30) Priority: 12.12.1996 EP 96120013; 30.05.1997 EP 97108723
(43) Date of publication of application: 06.10.1999
(73) Proprietor: Siegel, Günter, Prof. Dr. med., 12279 Berlin (DE); Malmsten, Martin, Dr., 117 30 Stockholm (SE)
(72) Inventor: Siegel, Günter, Prof. Dr. med., 12279 Berlin (DE); Malmsten, Martin, Dr., 117 30 Stockholm (SE)
(74) Representative: Lindner, Manfred Klaus, Dipl.-Phys.
(86) International application number: PCT/EP1997/005212
(87) International publication number: WO 1998/026292

(56) References cited:
- EP-A- 0 293 524
- WO-A-91/02975
- WO-A-97/17091
- US-A- 4 242 096
- US-A- 5 290 703

## Description

The present invention relates to a test substrate for mimicking atherosclerosis and arteriosclerosis and/or detecting atherosclerotic and arteriosclerotic risk, to a process for preparing said substrate, to a test system including said substrate and a method for detecting and testing atherosclerotic risk as well as effects of candidate drugs on the aforementioned.

Arteriosclerosis is a generic term for a number of diseases in which the arterial wall becomes thickened and loses elasticity. Atherosclerosis is the most important of these diseases. With its effects upon brain, heart, kidney, other vital organs and extremities, vascular disease is the leading cause of morbidity and mortality. Major risk factors of atherosclerosis are among others hypertension, elevated serum lipids such as elevated low density lipoprotein (LDL) and reduced levels of high density lipoprotein (HDL).

Atherosclerosis affects medium and large arteries and is characterized by patchy intramural thickening of the subintima that encroaches on the arterial lumen and in its most severe form causes obstruction. The atherosclerotic plaque consists of accumulation of intracellular and extracellular lipids, calcium salts, macrophages, smooth muscle cells, connective tissue and proteoglycans.

The earliest lesion of atherosclerosis is the fatty streak consisting of lipid-loaded foam cells, which are macrophages that have migrated as monocytes from the circulation into the subendothelial layer of the intima, which evolves into the fibrous plaque consisting of intimal smooth muscle cells surrounded by connective tissue and intracellular and extracellular lipid.

Atherosclerotic vessels have reduced systolic expansion and abnormally rapid pulse wave propagation. Sclerotic arteries of hypertensive subjects also have reduced elasticity, which is reduced further when atherosclerosis develops.

Two main hypotheses have been proposed to explain the pathogenesis of atherosclerosis. The lipid hypothesis postulates that an elevation of LDL results in penetration of LDL into the arterial wall, leading to lipid accumulation in smooth muscle cells and in macrophages (foam cells). LDL also augments smooth muscle cell hyperplasia in response to growth factors. LDL is oxidized to oxLDL in the presence of oxygen free radicals generated in the proximity of the membrane of the vascular wall cells, and acquires properties that render it more athecogenic. The chronic endothelial injury hypothesis postulates that endothelial injury produces loss of endothelium, adhesion of platelets to the subendothelium, aggregation of platelets, chemotaxis of monocytes and T-cell lymphocytes, and release of platelet- and monocyte-derived growth factors that induce migration of smooth muscle cells from the media into the intima.

The atherosclerotic plaque may grow slowly over years and produce severe stenosis or total occlusion. With time the plaque becomes calcified and may undergo spontaneous fissuring or rupture, with exposure of its contents to the flowing blood. The ruptured plaque stimulates thrombosis; the thrombi may embolize, rapidly occlude the lumen, or gradually become incorporated into the plaques, contributing to its bulk and occlusive properties.

The mechanism of flow-dependent dilatation of blood vessels is so far unknown. Indications, however, are growing that a sensor macromolecule anchored in the membrane of endothelial and smooth muscle cells as well as in the extracellular matrix initiates vascular relaxation.

Previous investigations have shown that electrolytes play an important role in the physicochemical behaviour of many macromolecular systems. In biological systems, electrolytes have been found to influence, e.g. self-association, macromolecular conformation, gel formation and adsorption. Electrolyte effects in biomacromolecular systems may be subdivided into three contributions of quite different physicochemical origins. These are the lyotropic effects, the general electrostatic effects and the specific ion-binding effects.

It has also been found that not only the above mentioned general electrostatic and non-specific lyotropic electrolyte effects but also specific ion-binding effects are important especially in many biological systems. The origin of these ion-specific effects varies between different systems but often they involve cations rather than anions.

One particular family of biological macromolecules which seems to display this sort of cation-specific ion binding is that of the proteoglycans. These are anionic biomacromolecules consisting of highly carboxylated and sulfated glycosaminoglycan chains covalently attached to a protein core. As such they are strong polyelectrolytes with a high linear charge density. The polyanionic and hydrophilic glycosaminoglycan chains dominate the physical properties of proteoglycans. They have a strong influence not only on tissue hydration and elasticity but also on counterion attraction by their negative charges. Examples of proteoglycans are proteoheparan sulfate, proteodermatan sulfate and proteochondroitin sulfate. A hybrid proteoheparan/chondroitin sulfate is incorporated into the membrane of endothelial cells in blood vessels via its hydrophobic domain consisting of the α-helical part of the protein core. The hydrophilic extracellular domain that protrudes into the blood solution is composed of a protein backbone to which glycosaminoglycan side chains are attached.

Since vascular occlusions, blood flow dysfunctions and the like, due to depositions of fatty substances such as cholesterol, calcium salts, macrophages, smooth muscle cells and connective tissue in atherosclerotic plaques within large and medium-sized arteries are irreversible processes, the early detection of these dysfunctions is subject to extensive investigations. Until now, no system or substrate for such a detection or recognition has been described in the prior art.

In view of the irreversibility of atherosclerotic plaque formation and its lethal side effects it was therefore an object of the present invention to provide a test system with which it is possible to detect risk of the vascular disease progression preferably in an early state.

In one aspect the present invention provides a process for preparing a test substrate for mimicking athero- and arteriosclerosis and/or detecting athero/arteriosclerotic risk.

In another aspect the invention provides a method for assaying the presence of atherosclerotic risk using the inventive test substrate.

Furthermore, methods are foreseen for recognizing risk for diseases of vascular occlusion, blood flow dysfunction and the like and thus provides the substrates within test systems for use in diagnostic tests for probing risk of atherosclerosis through monitoring lipoprotein deposition/binding and/or calcium-related binding/deposition as well as binding/deposition related to other cations at the substrate surface (developing a nomogram).

Furthermore, the invention comprehends methods for examination of drugs in a fast and cost effective manner that hinder lipoprotein deposition/binding and calcification.

It has been surprisingly found that surface modifications of hydrophobic substances, especially methylated silica surfaces by coupling with proteoheparan sulfate, lead to excellent results for mimicking athero- and arteriosclerosis in the vascular intima and media, sclerosis in basement membranes as well as sclerotic processes in matrices of tissues such as liver, pancreas, lung and the like. For this purpose, hydrophobic surfaces, in particular silica surfaces methylated through silane coupling, gold modified by thiol containing self assembling monolayers or any material modified through Langmuir-Blodgett or other deposition of lipophilic substances, e.g. mono-, di-, triglycerides, cholesterol or phospholipids or hydrophobic polymers, e.g. polystyrene, may be used. Furthermore, also inherently hydrophobic materials, e.g. plastics such as polystyrene, polyethylene, polycarbonate and the like, may be used as substrates for modifying the surface. These substrates are coupled with proteoheparan sulfate macromolecules.

The coupling of the above-mentioned surfaces with the above molecules is based on attachment from aqueous buffered blood substitute solution such as Krebs solution, modified Krebs solution and other saline aqueous solutions of physiological pH either with or without subsequent elimination of non-adsorbed macromolecules.

The above modified substrate will then be used in a method for assaying the presence of atherosclerotic risk from samples being whole or individual lipoprotein fractions from blood in which the serum proteins are removed and/or from other blood constituents with atherosclerotic risk potential, e.g. homocysteine, homocysteinic acid or from the whole serum. This method may comprise the steps of:
(a) preparing a modified surface of a suitable, especially hydrophobic compound;
(b) attaching a macromolecule with the hydrophobic surface;
(c) adding the sample being analysed, the lipoprotein binding/deposition and/or calcium-related binding/deposition and/or other cation-related binding/deposition can be detected by spectroscopic or microscopic analysis; and
(d) measuring the spectroscopic or microscopic response of lipoprotein deposition and/or calcium-related binding/deposition and/or other cation-related binding/deposition to indicate the atherosclerotic nature of the sample.

The sample to be analysed includes stratified endothelial cells, basement membranes and/or tissue matrices seleted from the group consisting of blood vessels, liver, pancreas, lung and the like.

Furthermore, it is possible to use the above modified substrate within the analysis method to investigate anti-atherosclerotic and anti-arteriosclerotic efficiency of drug formulations. This method may comprise the steps of:
(a) preparing a modified surface of a suitable, especially hydrophobic compound;
(b) attaching a macromolecule with the hydrophobic surface;
(c) adding the sample being analysed, the lipoprotein binding/deposition and/or calcium-related binding/deposition and/or other cation-related binding/deposition can be detected by spectroscopic or microscopic analysis; and
(d) measuring the spectroscopic or microscopic response of lipoprotein deposition and/or calcium-related binding/deposition and/or other cation-related binding/deposition to monitor the anti-athero/arteriosclerotic nature of the drug.

The inventive modified substrate is also useful for the investigation of atherosclerotic activity of any blood and tissue compounds.

The inventive test substrate may also used for the manufacture of a test system for mimicking athero/arteriosclerosis and/or detecting athero/arteriosclerotic risk, or for monitoring anti-athero/arteriosclerotic effects of a drug formulation.

The above analysis method may be carried out with any optical or spectroscopic method for investigating
- the adsorption of macromolecules at the surface or
- the adsorption of the sample at the surface or
- the adsorption of the sample at the test system, e.g. macromolecules adsorbed at the surface or
- the interfacial structure of the test system,
e.g. ellipsometry, reflectometry, surface plasmon resonance (SPR), total internal reflectance fluorescence spectroscopy (TIRF), Fourier transform infrared spectroscopy (FTIR). Also other methods for probing the binding/deposition related to calcium and/or other cations and/or lipoproteins at the systems are included such as atomic force microscopy and any type of microscopy. Generally, more extensive binding/deposition related to calcium and/or other cations and/or lipoproteins, processes which according to the above, can be mimicked with the use of the model, equals higher risk of atherosclerotic plaque formation.

It has been found that proteoglycans, e.g. heparan sulfate proteoglycan (HSPG) and dermatan sulfate proteoglycan (DSPG) adsorb at hydrophobic surfaces, for example methylated silica or mica hydrophobized through Langmuir-Blodgett deposition while the corresponding GAGs do not adsorb. Reproducible and well defined saturation adsorption values are obtained after less than two hours. Less than 10% of the adsorbed proteoglycans are desorbed on removal of the proteoglycan in solution after saturation adsorption. No adsorption occurs at hydrophilic and negatively charged surfaces, e.g. silica. Thus adsorption at hydrophobic surfaces is driven by a hydrophobic interaction between the surface and the protein moiety while GAG chains are oriented towards the aqueous solution as also indicated directly with surface force measurements.

It has also been found that analogously to the behaviour of proteoglycans in their biological environment, the surface-attached proteoglycans interact with electrolytes in a strongly ion-specific manner. In particular Na⁺ and Ca²⁺ were found to display this interaction and control both the adsorption and the interfacial conformation of the adsorbed proteoglycans. Parallel experiments on the regulation of vascular tone show that these ions were found to be particularly important also in isolated human vascular strips thus supporting the biological relevance of the test system.

The electrolyte-dependent adsorption can be modulated by addition of certain compounds. In particular the Ca²⁺-induced increased proteoglycan adsorption as well as an interfacial conformational change can be reduced by a known anti-atherosclerotic drug formulation, e.g. garlic extract, containing active components such as allicin and ajoene, which by parallel experiments was found due to, at least partly, a reduced proteoglycan-calcium binding. Therefore, the effects of various compounds on the calcium binding to the respective macromolecule in the biological system may be simulated.

Known risk factors for atherosclerosis like homocysteine were found to interact with the test system and strongly modify in particular the calcium interaction with the test system.

Lipoproteins, e.g. LDL, oxLDL and Lp(a) were found to interact with the test system and to form an interfacial deposition in a highly species-dependent manner. The test system thus allows fast and cost effective *in vitro* studies of lipid deposition at its natural binding site at the endothelial cell membrane and in the tissue matrix, a key feature and a potent initiator of atherosclerosis.

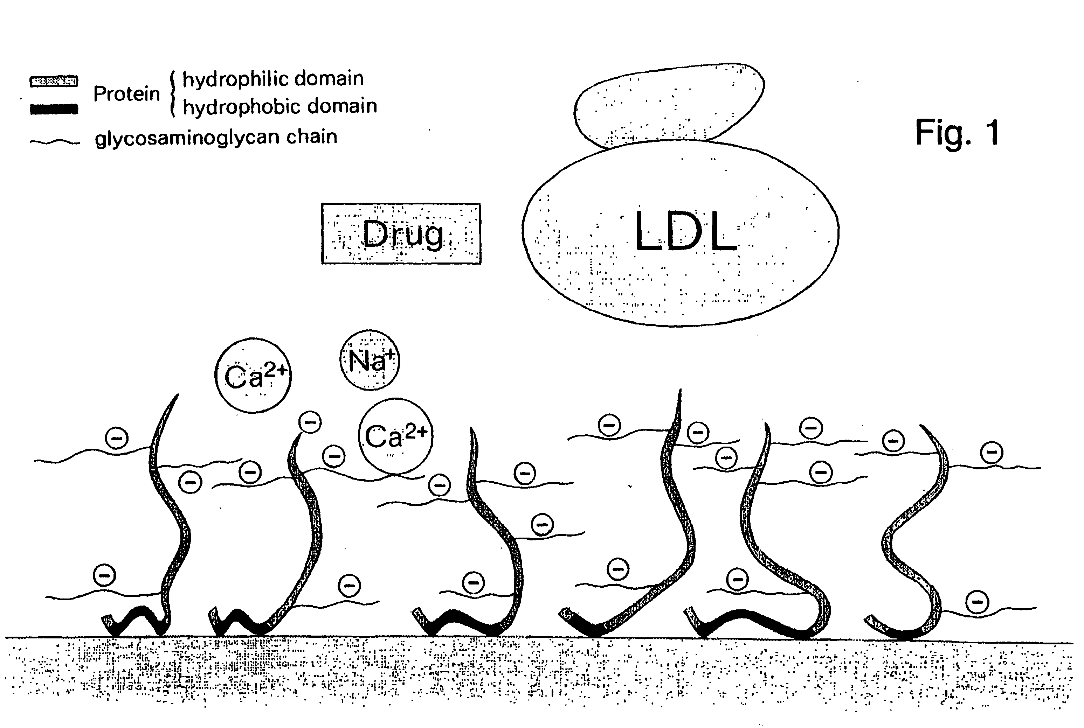

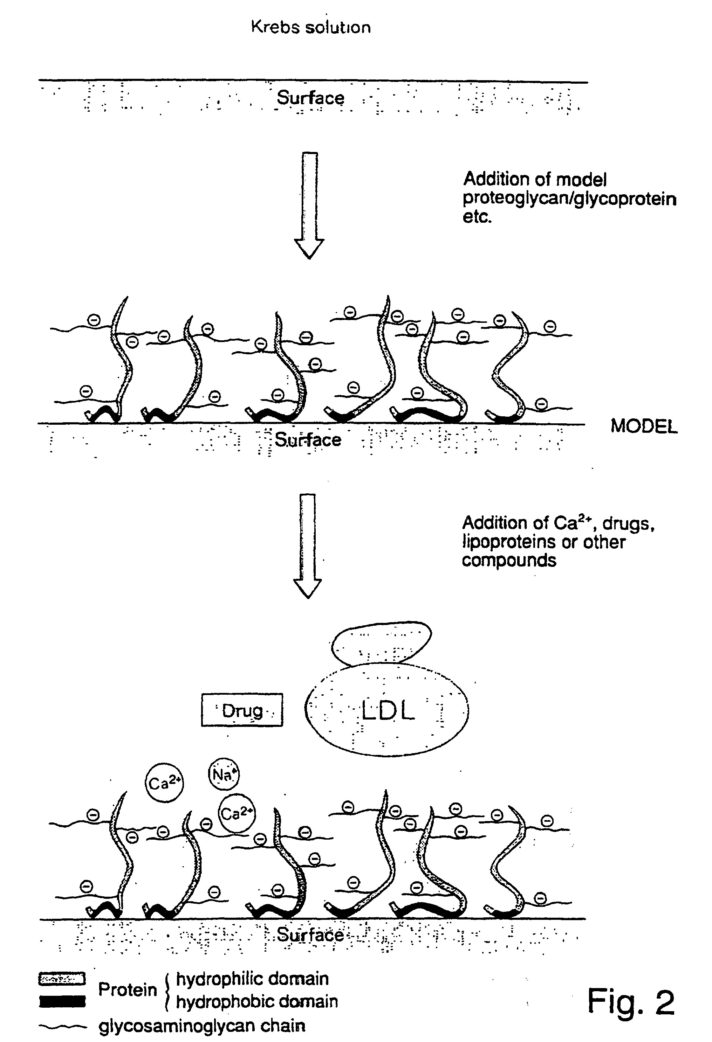

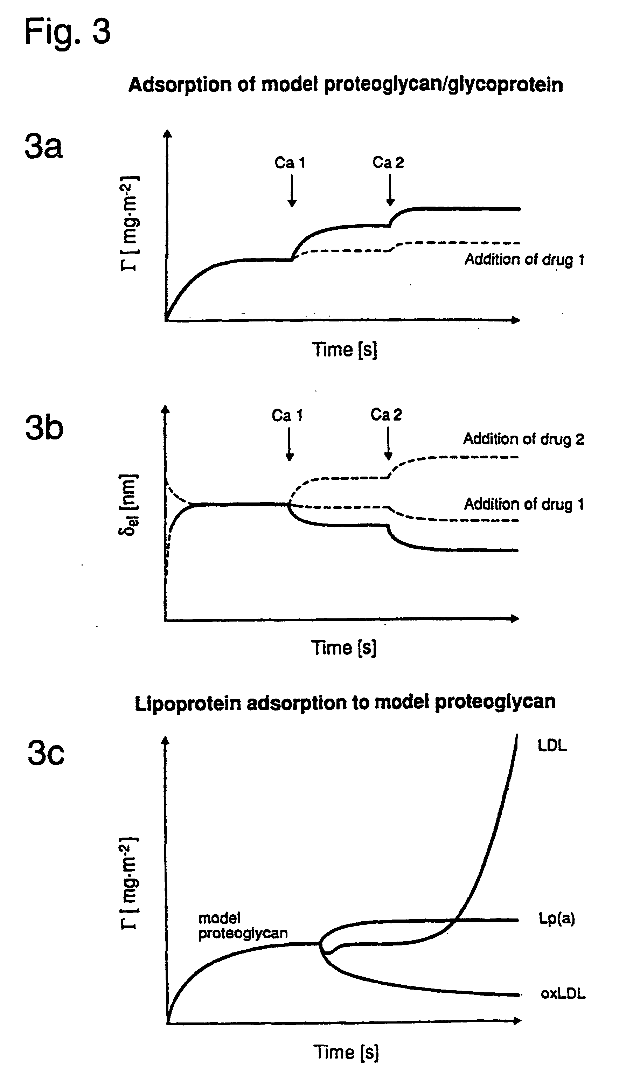

The advantageous findings using the inventive substrate and method are shown in figs. 1 and 2 as well as in fig. 3a, 3b and 3c, in which Γ represents the total adsorbed amount [mg/m²] in the presence of two calcium concentrations Ca1 and Ca2 (fig. 3a) as well as in the presence of anti-atherosclerotic drugs 1 and 2 (fig. 3b) and in the presence of lipoproteins (fig. 3c).

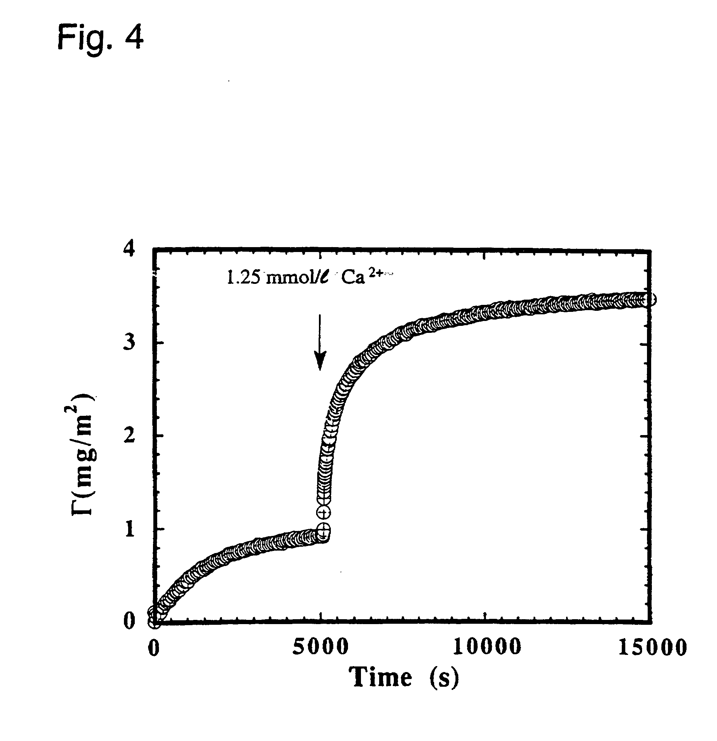

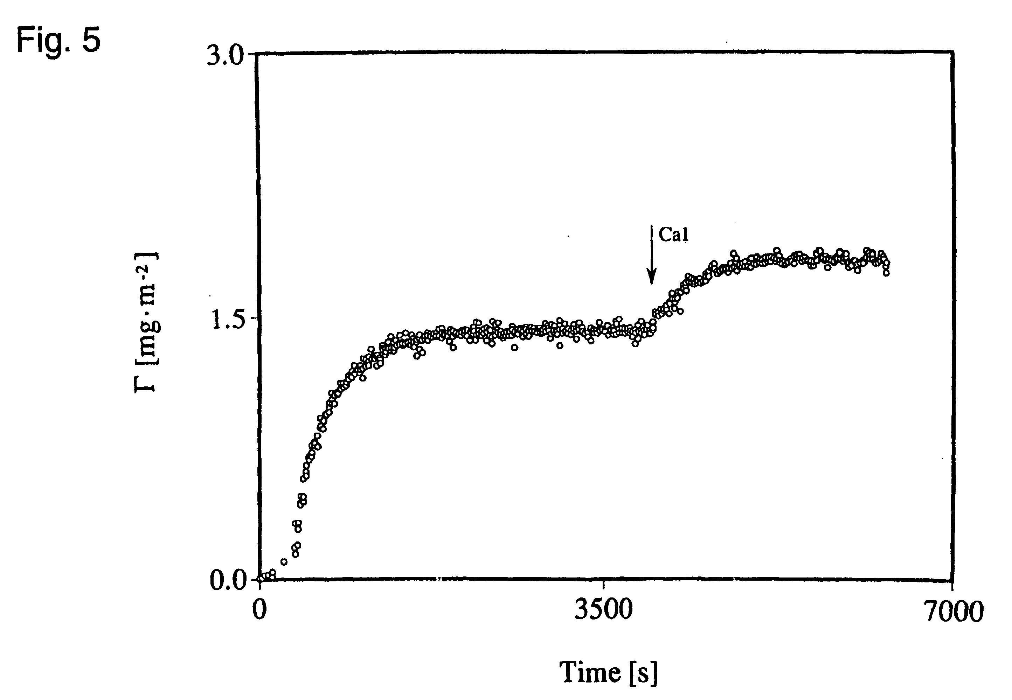

The invention will be described in more detail with reference to the figures 4 to 14 being non-limitative for the scope of the invention. The detailed description of the procedure for modifying the substrate and the test system are shown below (see examples).

Figs. 4 (pure water) and 5 (Krebs solution) show the proteoheparan sulfate deposition at the methylated silica surface by its transmembrane hydrophobic core domain. The adsorbed amount is depicted on the ordinate. Since Ca²⁺ ions screen the negative glycosaminoglycan chains, this ion species promotes proteoheparan sulfate adsorption even with a physiological Ca²⁺ concentration of only 1.25 mmol/l.

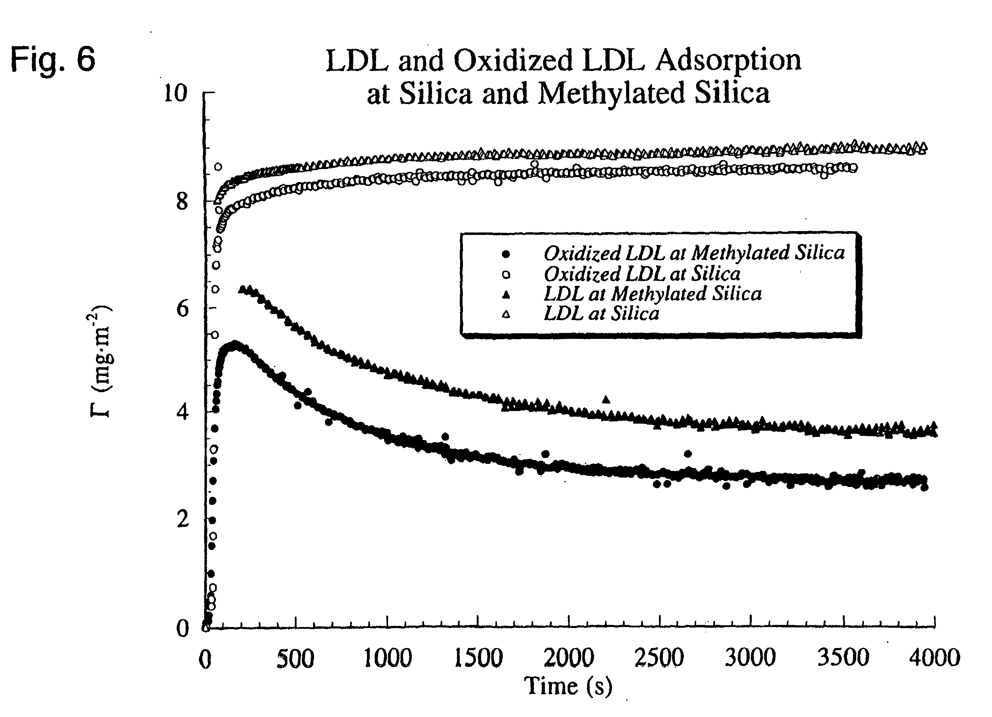

Fig. 6 is a graphical illustration of the LDL and oxLDL adsorption at silica and methylated silica exhibiting a quite different time course. It can be seen that, for methylated silica, after a fast initial adsorption a desorption period of approximately one hour follows. After that time the adsorption was constant.

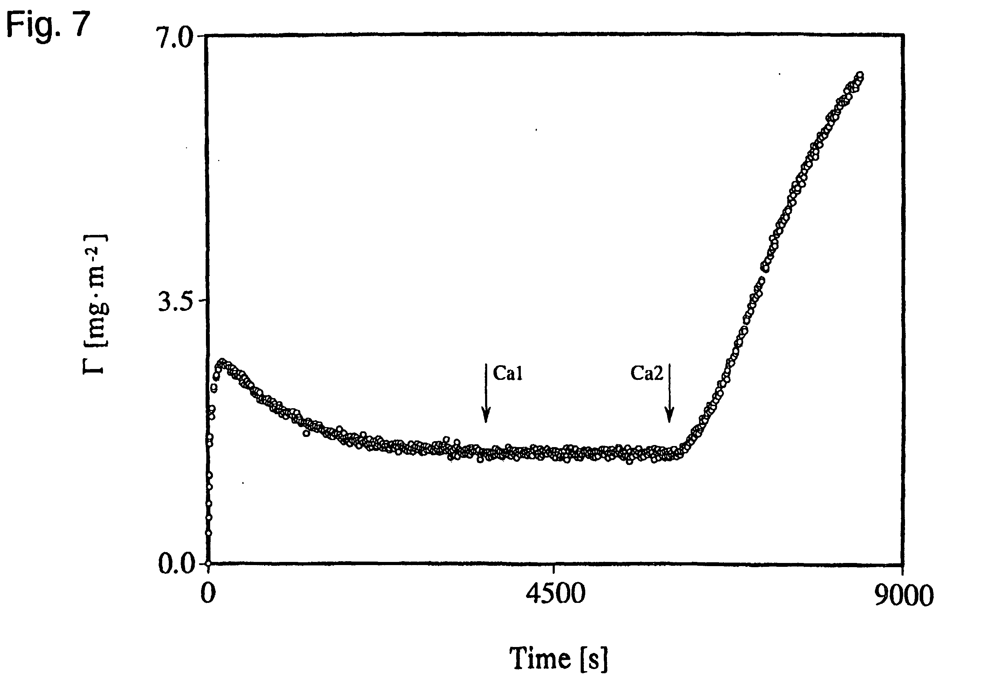

Fig. 7 shows the investigation of whether Ca²⁺ ions influence the LDL adsorption from a Ca²⁺-free Krebs solution as could be demonstrated for proteoheparan sulfate. It can be seen that Ca1 (2.5 mmol/l) does not change or, in not-shown experiments, slightly increases the LDL adsorption, whereas Ca2 (10 mmol/l) accelerates the adsorption dramatically, probably by a mechanism related to aggregation.

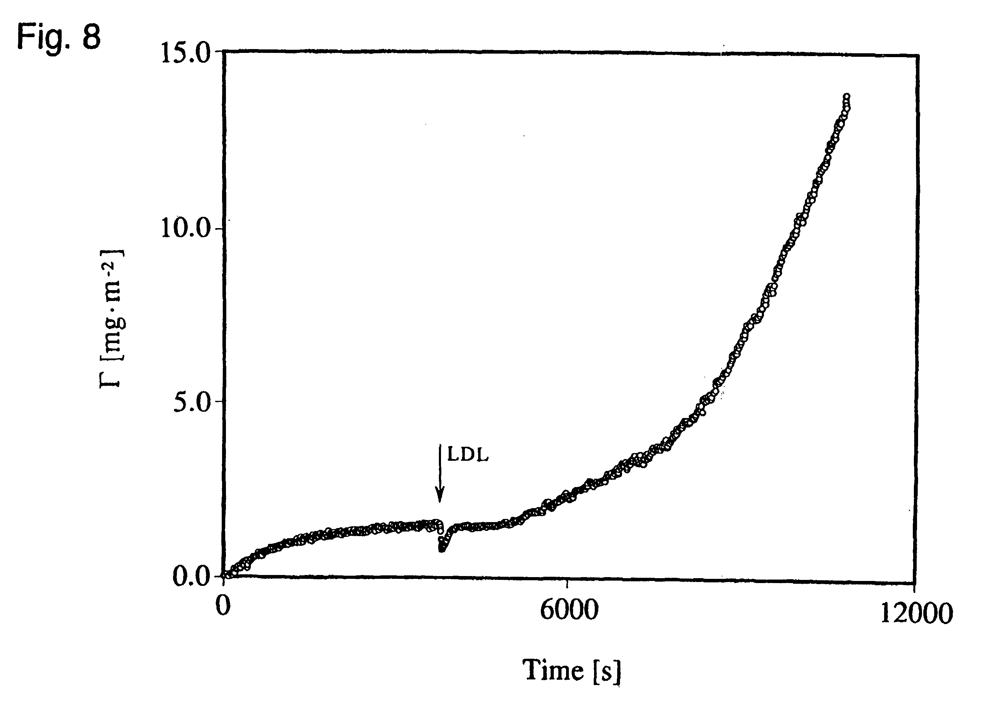

Fig. 8 is a diagram depicting a simulation of the physiological scenario. Lipoprotein binding to heparan sulfate proteoglycan preadsorbed from a Ca²⁺-free Krebs solution at methylated silica resulted in no additional adsorbed amount for LDL during the first 25 minutes; thereafter with LDL aggregation the adsorbance increased to 14 mg/m² within 95 minutes.

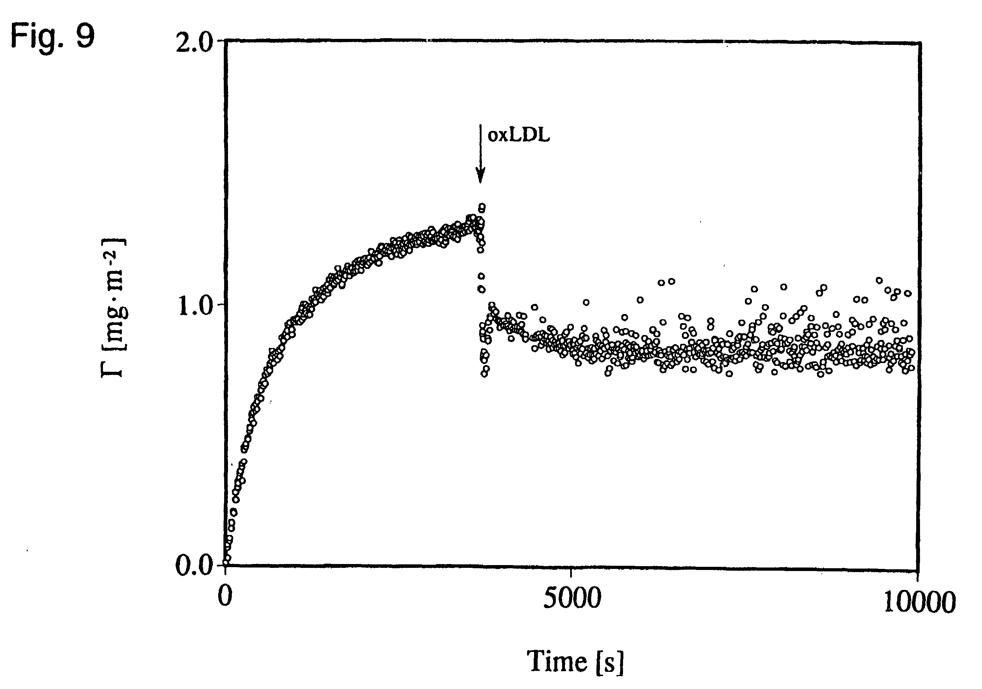

Fig. 9 shows in contrast that oxLDL steadily reduced the adsorbed amount of the proteoheparan sulfate/oxLDL complex to 0.65 mg/m² within 2 hours. Thus oxLDL seems to bind strongly to proteoheparan sulfate and to alter its polyanionic glycosaminoglycan binding sites possibly by the intervention of oxygen free radicals.

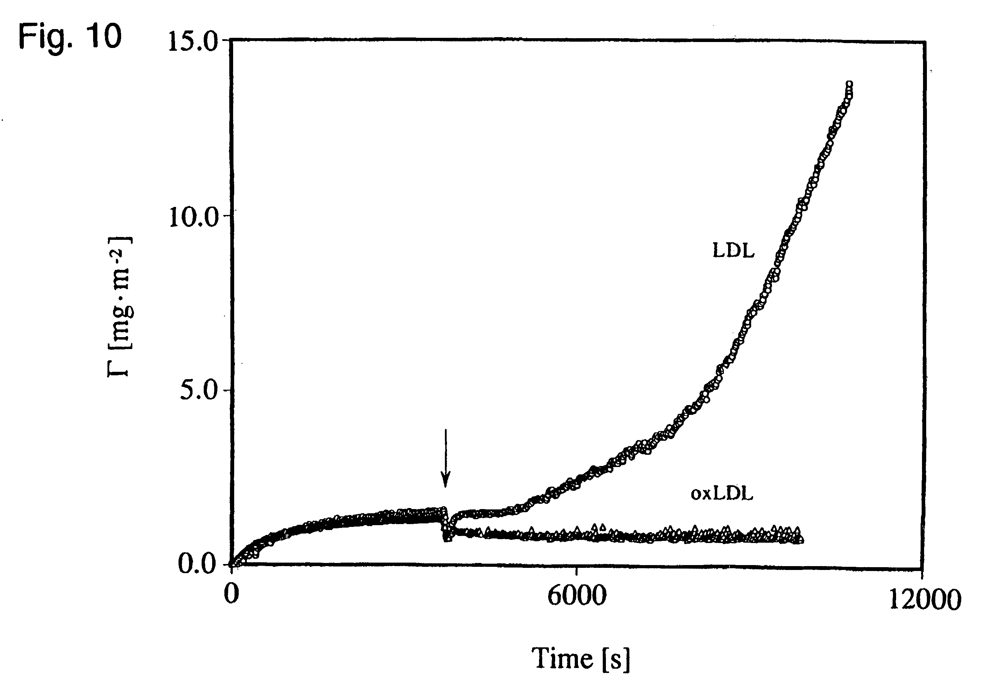

Fig. 10 summarizes the results of the measurements depicted in figs. 8 and 9. It can be seen that LDL and oxLDL binding to preadsorbed proteoheparan sulfate exhibits a large divergence. While LDL strongly increases the adsorbance, oxLDL diminishes the amount of the adsorbed proteoheparan sulfate/oxLDL complex.

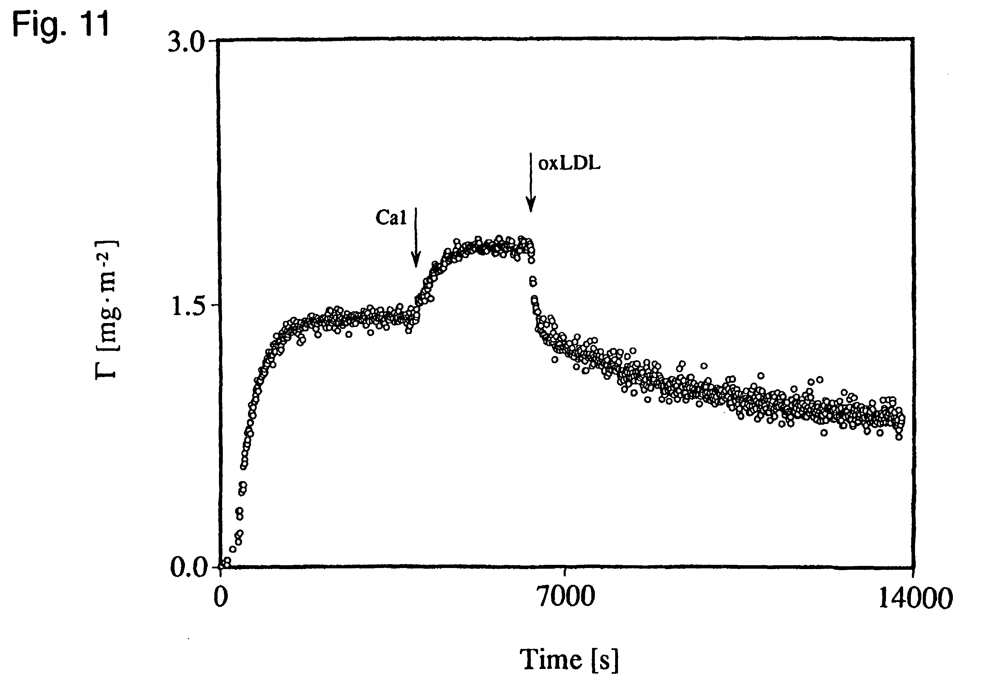

Fig. 11 shows this aggressive effect of oxLDL. Proteoheparan sulfate adsorbance from Ca²⁺-free Krebs solution was strongly pronounced by the addition of Ca²⁺ ions thus screening the electrostatic interplay which counteracts adsorption. The intervention of oxLDL initiated an immediate desorption that proceeded progressively over several hours.

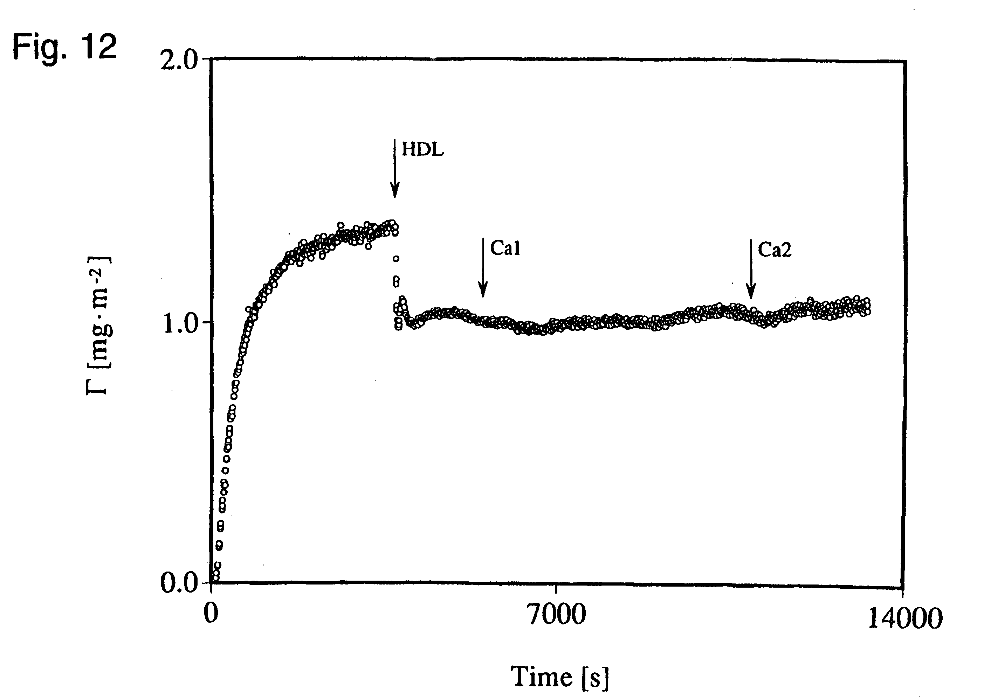

Fig. 12 demonstrates an experiment to elucidate how the above mentioned effects are modified by calcium ions and if a calcification of the proteoglycan/lipoprotein complex takes place. HDL (high density lipoprotein) binding to heparan sulfate proteoglycan was only slightly increased by stepwise additions of Ca²⁺ ions. Furthermore, HDL seems to strongly bind to heparan sulfate proteoglycan.

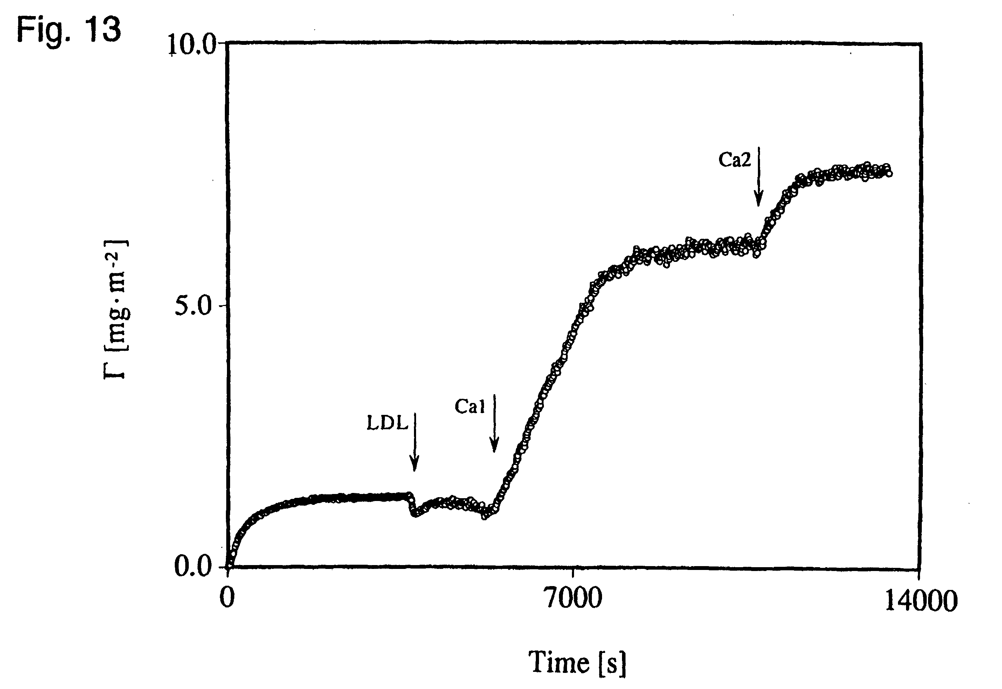

Fig. 13 shows in contrast to the results of the experiments demonstrated in fig. 12 that Ca²⁺ ions dramatically changed the proteoheparan sulfate/LDL interaction. After preadsorption of proteoheparan sulfate, LDL did not significantly alter the adsorbed amount within the observation time frame. However, the addition of 2.5 mmol/l Ca²⁺ ions promotes the proteoheparan sulfate/LDL interaction.

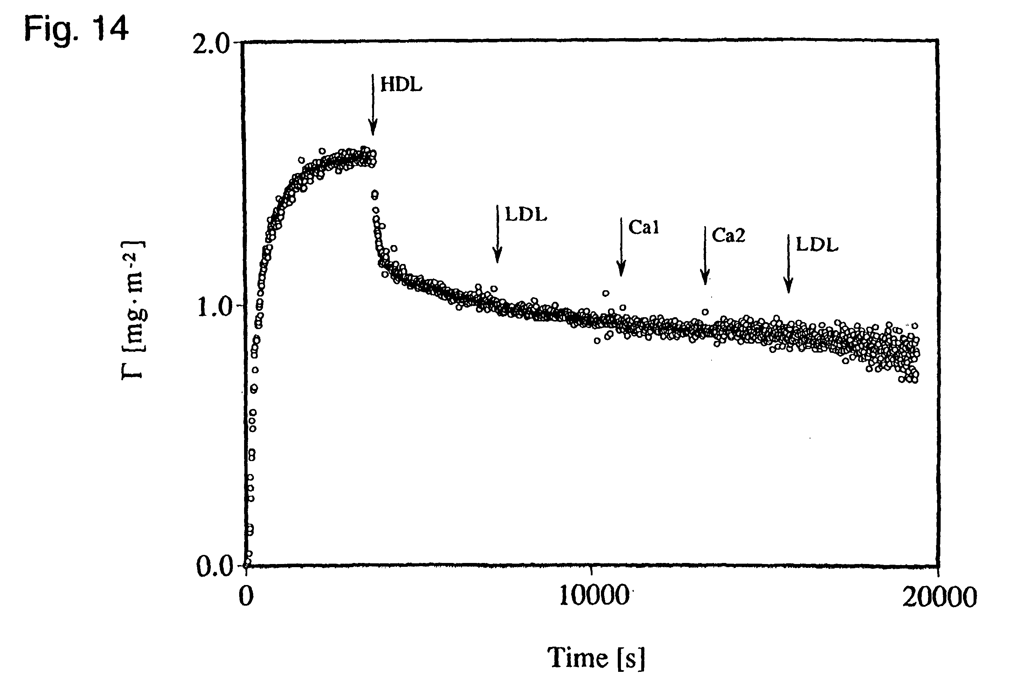

Further investigations were designed to examine the protective role of HDL. The results are shown in fig. 14. After proteoheparan sulfate adsorption from a Ca²⁺-free Krebs solution, HDL induced a desorption which was not affected by the addition of LDL in physiological concentrations. Most surprisingly it was found that Ca²⁺ ions had no effect at all, even at a concentration of 10 mmol/l. Moreover, neither did the repeated addition of LDL (double serum concentration) change the adsorption.

The experiments indicate that HDL binds with high affinity to heparan sulfate proteoglycan and excludes LDL from its electrostatic attraction to the polyanionic proteoglycans. Furthermore, the 'calcification' of the proteoheparan sulfate/LDL complex seems to be inhibited. Therefore, this result underscores the direct anti-atherosclerotic and anti-arteriosclerotic effect of high density lipoprotein.

Furthermore, it is now possible with the inventive test system to reduce the high cost and long development time of new candidate drugs against athero- and arteriosclerosis due to extensive animal testing. In view of the irreversibility of atherosclerosis with the known side effects of existing anti-atherosclerotic drugs, the test system can provide information for an early detection of risk for developing atherosclerosis. Moreover, the test system can provide a research tool for investigations of molecular mechanisms of athero-/arteriosclerosis as well as the effects of anti-athero/arteriosclerotic drugs.

In the following, the methods for substrate modification are described in more detail and it has to be mentioned that the invention is not limited to these methods.

### Examples

### Materials

Ultra pure water should be used in the experiments. Therefore, water was first purified by a Milli-RO 10PLUS pretreatment unit, including depth filtration, carbon adsorption and decalcination preceding reverse osmosis. Subsequently, it was led through a Milli-Q PLUS 185 unit, which treats the feed water with UV light (185 nm and 254 nm) before leading it into a Q-PAK unit consisting of an active carbon unit followed by a mixed bed ion exchanger, an Organex cartridge, and a final 0.22 mm Millipak 40 filter. However, also other water purification methods might be acceptable.

Proteoheparan sulfate (Na⁺ salt) was prepared as previously described by Schmidt, A., Schäfer, E., Buddecke, E. (1988): Isolation and characterization of two proteoheparan sulfate species of calf arterial tissue, Eur. J. Biochem. 173, 661-666. This biomolecule had an average molecular weight of 175 kD and is a highly negatively charged macromolecule [95% of all molecules lie within the range of 165-185 kD]. It contains a protein core (Mᵣ = 38 kD) to which a few heparan sulfate side chains (Mᵣ ≈ 35 kD) are covalently linked. The heparan sulfate side chains, which may be obtained from proteoheparan sulfate by exhaustive proteolytic digestion or by a β-elimination reaction, consist of repeating uronic acid (1-4) glucosamine disaccharides, but exhibit a broad chemical and configurational variability with respect to the ratio of glucuronic acid/iduronic acid, the number and position of *O*-sulfate ester groups and the ratio of *N*-sulfate/*O*-sulfate. One disaccharide unit contains 1 carboxyl and 0.5 sulfate groups on average. Proteodermatan/-chondroitin sulfate was prepared as described by Schmidt, A., Prager, M., Selmke, P., Buddecke, E. (1982): Isolation and properties of proteoglycans from bovine aorta, Eur. J. Biochem. 125, 95-101. This proteoglycan has a Mᵣ of 190 000 and contains 23% protein (Mᵣ 46 000) with 3-4 hybrid chains (Mᵣ 39 000 ± 4 200) covalently linked to the protein core by alkali labile bonds. The chondroitin sulfate/dermatan sulfate side chains have a copolymeric structure and consist of 53% chondroitin sulfate and 47% dermatan sulfate with an *O*-sulfate ester content of 0.96 sulfate groups and one carboxyl group per disaccharide unit. Note, however, that also other proteoglycan preparations may be acceptable for the sensor.

The electrolytes were all of analytical grade, and used without further purification. Note, that also other electrolyte qualities may be acceptable.

### Surfaces

Silica surfaces were obtained from polished silicon slides. In short, these were oxidized thermally in oxygen, followed by annealing and cooling in argon flow, which resulted in an oxide layer thickness of about 30 nm. The slides were then cleaned in a mixture of 25% NH₄OH, 30% H₂O₂ and H₂O (1:1:5, by volume) at 80°C for 5 min, followed by cleaning in a mixture of 32% HCl, 30% H₂O₂ and H₂O (1:1:5, by volume) at 80°C for 5 min. The slides were then rinsed twice with, in order, water, ethanol and trichloroethylene (pro analysi, Merck), followed by a treatment with a 0.1 wt% solution of Cl₂(CH₃)₂Si (Merck) in trichloroethylene for 90 minutes. Finally, they were rinsed again four times in trichloroethylene and ethanol. This procedure rendered the slides hydrophobic, with an advancing and receding contact angle of 95° and 88°, respectively. They were then kept in ethanol until use. Note, however, that a range of different surfaces and surface modifications may be used, depending on the technique employed.

### Methods

On the basis of ellipsometry measurements the procedure is exemplarily explained. It has to be noted that all above mentioned spectroscopy and microscopy methods can be used.

The ellipsometry measurements were all performed by means of null ellipsometry as described by Azzam, R.M.A., Bashara, N.M. (1989) Ellipsometry and polarized light, North-Holland, Amsterdam. The instrument used was an automated Rudolph thin-film ellipsometer, type 436, controlled by a personal computer. A xenon lamp, filtered to 4015 Å, was used as the light source. A thorough description of the experimental setup is given in Malmsten, M., Siegel, G. (1995) Electrostatic and ion-binding effects on the adsorption of proteoglycans. J. Colloid Interface Sci. **170**, 120-127. All measurements were performed by four-zone null ellipsometry in order to reduce effects of optical component imperfections. After the optical analysis of the surface, the proteoglycan solution was added to the cuvette, and the polarization changes were recorded. The maximal time resolution between two measurements is 3-4 seconds. Finally, the electrolyte composition and/or the lipoprotein concentration of the bulk solution were varied either with or without preceding rinsing to remove non-adsorbed molecules. Throughout, corrections were made for changes in the bulk solution refractive index on changing the composition. Stirring was performed by a magnetic stirrer at about 300 rpm.

The adsorbed amount (Γ) was obtained from the determined adsorbed layer thickness and mean refractive index data, using a value of dn/dc of 0.16 cm³/g for both proteoheparan sulfate and proteodermatan/-chondroitin sulfate. All measurements were performed with a constant bulk proteoglycan concentration of 0.1 mg/ml (corresponding to plateau adsorption). Furthermore, the measurements were performed at different electrolyte concentrations around those in the biological system. The normal blood substitute solution (Krebs solution) consisted of: Na⁺ 151.16; K⁺ 4.69; Ca²⁺ 2.52; Mg²⁺ 1.1; Cl⁻ 145.4; HCO₃⁻ 16.31 and H₂PO₄⁻ 1.38 mmol/l. Throughout, the pH was kept at 7.24 ± 0.01 by the bicarbonate/phosphate buffer, and by a continuous aeration of the cuvette solution with a 95% O₂ - 5% CO₂ or 94% N₂ - 6% CO₂ gas mixture (Aga, Sweden). The latter are included due to the necessity to keep the pH well regulated throughout these experiments, sometimes at non-oxidizing conditions, in order to avoid both degradation and calcium phosphate precipitation. It has to be mentioned that also other procedures may well be acceptable for the sensor application.

## Claims

1. A substrate for mimicking athero/arteriosclerosis and/or detecting athero/arteriosclerotic risk being composed of a modified surface of a suitable, hydrophobic substrate, coupled with a proteoheparan sulfate macromolecule.

2. Method for making the substrate according to claim 1, comprising preparing the surface and coupling proteoheparan sulfate macromolecule to said surface.

3. Method according to claim 2, **characterized in that** said macromolecule is coupled to the surface by adsorbing said macromolecule to said surface.

4. A method to assaying for the presence of atherosclerotic risk in a blood sample by at least one of spectroscopic and microscopic analyses, comprising: i) preparing a methylated silica surface, ii) coupling proteoheparan sulfate to said surface, iii) adding said sample to said coupled proteoheparan sulfate, and iv) measuring at least one spectroscopic or microscopic response of the deposition of lipoprotein components of the blood sample to said coupled macromolecule.

5. A method for detecting anti-atherosclerotic efficiency of a drug by at least one of spectroscopic and microscopic analyses, comprising: i) preparing a methylated silica surface, ii) coupling proteoheparan sulfate to said surface, iii) adding the drug being analysed to said coupled macromolecule, and iv) measuring the spectroscopic or microscopic response of lipoprotein deposition.

## Patentansprüche

1. Substrat zum Nachahmen von Athero/Arteriosklerose und/oder Detektieren von Athero/Arterioskleroserisiko, bestehend aus einer modifizierten Oberfläche eines geeigneten hydrophoben Substrats, gekoppelt mit einem Proteoheparansulfat-Makromolekül.

2. Verfahren zum Herstellen des Substrats nach Anspruch 1, enthaltend das Präparieren der Oberfläche und Koppeln eines Proteoheparansulfat-Makromoleküls an die Oberfläche.

3. Verfahren nach Anspruch 2, **dadurch gekennzeichnet, dass** das Makromolekül an die Oberfläche durch Adsorbieren des Makromoleküls an der Oberfläche gekoppelt wird.

4. Verfahren zum Untersuchen der Präsenz eines atherosklerotischen Risikos in einer Blutprobe durch wenigstens eine spektroskopische oder eine mikroskopische Analyse, enthaltend: i) Präparieren einer methylierten Silikaoberfläche, ii) Koppeln von Proteoheparansulfat an die Oberfläche, iii) Hinzufügen der Probe zu dem gekoppelten Proteoheparansulfat, und iv) Messen wenigstens eines spektroskopischen oder mikroskopischen Responses der Deposition von Lipoproteinkomponenten der Blutprobe auf das gekoppelte Makromolekül.

5. Verfahren zum Detektieren einer anti-atherosklerotischen Effizienz eines Arzneimittels durch wenigstens eine spektroskopische oder mikroskopische Analyse, enthaltend: i) Präparieren einer methylisierten Silikaoberfläche, ii) Koppeln von Proteoheparansulfat an die Oberfläche, iii) Hinzufügen des analysierten Arzneimittels zu dem gekoppelten Makromolekül, und iv) Messen des spektroskopischen oder mikroskopischen Responses der Lipoproteindeposition.

## Revendications

1. Substrat pour simuler une athérosclérose/artériosclérose et/ou pour détecter un risque athérosclérotique/artériosclérotique, composé d'une surface modifiée d'un substrat hydrophobe approprié, couplé avec une macromolécule de sulfate de protéohéparane.

2. Procédé pour réaliser le substrat selon la revendication 1, comprenant la préparation de la surface et le couplage d'une macromolécule de sulfate de protéohéparane à ladite surface.

3. Procédé selon la revendication 2, **caractérisé en ce que** ladite macromolécule est couplée à la surface par adsorption de ladite macromolécule sur ladite surface.

4. Procédé d'essai relatif à la présence de risque athérosclérotique dans échantillon sanguin à l'aide d'une analyse au moins parmi l'analyse spectroscopique et l'analyse microscopique, comprenant : i) la préparation d'une surface de silice méthylée, ii) le couplage de sulfate de protéohéparane sur ladite surface, iii) l'addition dudit échantillon audit sulfate de protéohéparane couplé, et iv) la mesure d'au moins une réponse spectroscopique ou microscopique de la déposition de composants lipoprotéines de l'échantillon sanguin sur ladite macromolécule couplée.

5. Procédé pour détecter l'efficacité anti-athérosclérotique d'un médicament à l'aide d'une analyse au moins parmi l'analyse spectroscopique et l'analyse microscopique, comprenant : i) la préparation d'une surface de silice méthylée, ii) le couplage de sulfate de protéohéparane sur ladite surface, iii) l'addition du médicament à analyser à ladite macromolécule couplée, et iv) la mesure de la réponse spectroscopique ou microscopique de la déposition de lipoprotéine.
